# EUROPEAN PATENT SPECIFICATION

(11) **EP 1 288 306 B1**
(45) Date of publication and mention of the grant of the patent: **21.10.2009**
(21) Application number: 01934540.4
(22) Date of filing: 05.06.2001
(51) Int. Cl.: C12Q 1/44, G01N 33/92

(54) **METHOD OF ANALYZING COMPONENTS IN BIOLOGICAL SAMPLES**
VERFAHREN ZUR ANALYSE VON KOMPONENTEN BIOLOGISCHER PROBEN
METHODE D'ANALYSE DE COMPOSANTS DANS DES ECHANTILLONS BIOLOGIQUES

(30) Priority: 07.06.2000 JP 2000171136
(43) Date of publication of application: 05.03.2003
(73) Proprietor: SYSMEX CORPORATION, Kobe-shi, Hyogo 651-0073 (JP)
(72) Inventor: KISHI, Koji, Kobe-shi, Hyogo 651-2241 (JP); KAKUYAMA, Tsutomu, Kobe-shi, Hyogo 651-2241 (JP); OCHIAI, Koji, Kobe-shi, Hyogo 651-2241 (JP)
(74) Representative: Krauss, Jan
(86) International application number: PCT/JP2001/004721
(87) International publication number: WO 2001/094619

(56) References cited:
- WO-A1-98/59068
- JP-A- 9 043 234
- JP-A- 11 009 300
- US-A- 6 114 134
- STN CAPLUS, vol. 131, no. 4, 26 July 1999 (1999-07-26), XP002144468
- NAUCK M ET AL: "ANALYTICAL PERFORMANCE AND CLINICAL EFFICACY OF THREE ROUTINE PROCEDURES FOR LDL CHOLESTEROL MEASUREMENT COMPARED WITH THE ULTRACENTRIFUGATION-DEXTRAN SULFATE-MG2+ METHOD" CLINICA CHIMICA ACTA, AMSTERDAM, NL, vol. 294, April 2000 (2000-04), pages 77-92, XP000985507 ISSN: 0009-8981

## Description

### Technical Field

The present invention relates to a method and a reagent kit for the testing of components in very low-density lipoprotein (VLDL) in blood. More particularly, it relates to a method and a reagent kit for the selective assay of cholesterol in VLDL.

### Background Art

Regarding lipoprotein, serum contains four kinds of lipoprotein, i.e. high-density lipoprotein (HDL), low-density lipoprotein (LDL), very low-density lipoprotein (VLDL) and chylomicron (CM). It has been known that diseases such as various types of hyperlipemia are derived from an increase in one or more of such lipoprotein fractions, and methods for a quantitative determination by fractionation into each lipoprotein have been reported.

Fractionation of lipoprotein has been carried out by means of old-fashioned ultracentrifugation. This method requires skillfulness and is conducted by installing separate ultracentrifuge and centrifugation is carried out for several days. Therefore, it was not possible to treat many samples. Besides this, there is a method where lipoprotein is separated by electrophoresis, the amount of protein is determined, and a method for the testing of cholesterol for each lipoprotein by HPLC is performed, but this method lacks processing capacity and, in addition, expensive apparatuses in addition to the common automatic analysis apparatus are necessary.

Recently, in order to solve the above-mentioned problem concerning the testing of cholesterol in HDL, a fully automatic kit for the testing of cholesterol in HDL has been developed and is commercially available. The state of the art mentioned in Japanese Patent No. 2,600,065 (November 30, 1994), Japanese Patent Laid-Open No. 08/201,393 (January 31, 1995) and Japanese Patent Laid-Open No. 08/131,195 (December 21, 1994) uses a fractionating agent, but the metal used as divalent cation contained in the fractionating agent forms an insoluble precipitate with the detergent that is commonly used in an full-automatic analyzing apparatus, and accumulates as a liquid waste causing problems. In addition, an insoluble aggregate is formed during the reaction resulting in turbidity which affects the assayed results and causes testing errors and, further, the reaction cell is polluted by the aggregate which significantly affects the testing results for other biochemical parameters being assayed at the same time.

There are many cases where the reaction is completed within 10 minutes in most of the common automatic analyzing apparatuses. Moreover, it is possible to select between the two-point end method, the rate method, the double rate method, the fix time method, etc. which are known methods and, therefore, the assay is possible even in a turbid state. However, such an assay in a turbid state causes an error in the assayed data if there are changes in turbidity during the reaction, and thus there is a problem with precision. In addition, reproducibility is reduced when the reaction solution becomes turbid. Therefore, there is a limitation in the sample to be assayed and it is not possible to use broad assaying wavelengths and various kinds of samples of patients. For example, near 340 nm (UV region), there is a disadvantage that absorbance becomes 2∼3 or more due to a turbidity phenomenon caused by the aggregate and is sometimes found outside of the allowable range of the analyzer. Japanese Patent Laid-Open No. 09/96,637 (July 19, 1996) describes a method where no divalent cation is used comprising anti-serum aggregating with lipoprotein but even this method forms a hardly soluble antigen-antibody aggregate and, therefore, the reaction cell is polluted. Accordingly, this greatly affects the assayed data for other biochemical parameters assayed at the same time. Further, since the turbidity in the reaction solution becomes high, for the same reason as above a precise assay for cholesterol in HDL is impossible, especially in the UV region.

This art comprises a device for an assaying method and a common technique for inhibiting the enzymatic reaction through the formation of complexes and aggregates, but the bad effects inherent to the turbidity are not overcome.

With regard to the assay of cholesterol in LDL (LDL-C), there are reports for an assay method of LDL-C with the object of full automation, as noted in Japanese Patent Laid-Open No. 07/280,812 (April 5, 1994), WO 96/29599 (March 20, 1995) and Japanese Patent Laid-Open No. 09/313,200 (May 29, 1996). Like the assay of cholesterol in HDL (HDL-C), these assaying methods are on the extension line of the above-mentioned art forming aggregate and complex and, therefore, it remains a matter to be resolved in the future, how to handle the turbidity.

On the other hand, in the testing and diagnosis of lipoprotein-related diseases such as hyperlipemia, information only concerning HDL-C and LDL-C is insufficient as clinical information. The World Health Organization (WHO) classifies hyperlipemia into five types [Rinsho Kensa, Vol. 40, No. 9, page 107 (1996)] in which the fate of cholesterol in VLDL (VLDL-C) is included as well. Thus, for example, in type IIa of hyperlipemia II, LDL increases whereas, in the type IIb, LDL and VLDL increase. In the type IV, VLDL decreases whereas, in the type V, VLDL increases. As such, hyperlipemia can not be determined by the fate of a single lipoprotein. Further, in the study concerning lipoprotein metabolism in coronary artery diseases such as arteriosclerosis caused by hyperlipemia, information on VLDL-C has also become necessary. Furthermore, there is a report that the fate of remnant-like lipoprotein (RLP) is a direct trigger for arteriosclerosis [McNamara J. R., et al., Clin. Chem., 44, 1224-1232 (1998)]. Testing of not only VLDL-C but also VLDL sub-fractions such as RLP-C is being regarded as important. Anyway, there is no assaying method where an operation for the separation of VLDL-C is unnecessary and, at present, there is no way but to rely on ultracentrifugation, electrophoresis, HPLC, etc. Accordingly, this is a reason for no progress in the study despite of its clinical significance.

EP 1020232 describes a method for assaying cholesterol in VLDL where in the first reaction cholesterol in HDL and LDL is tested, wherein VLDL is made stable by forming VLDL-calixarene complexes, and in the second reaction cholesterol is tested after decomposition/release from the complex, and then said cholesterol in VLDL is analysed based on the differences between the result of the first reaction and the result of the second reaction. In contrast, in the method of the present invention, the whole method is carried out in the presence of calixarene, albumin or the basic amino acid. The test for cholesterol in VLDL is performed in that the reactivity of LDL and enzyme is suppressed by using calixarene, and the reactivity of the HDL and enzyme is suppressed by using albumin or a basic amino acid.

STN CAPLUS, vol. 131, No. 4, discloses the general concept that calixarene transforms lipoproteins inhuman serum into complexes, and that the complexes react differently with cholesterol hydroxylase.

JP 11 009 300 describes a method for testing cholesterol in LDL, wherein a reactivity of HDL and VLDL with an enzyme is suppressed by using albumin. However, JP 11 009 300 does not describe an assay method for cholesterol in VLDL. Furthermore, a test for cholesterol in VLDL, wherein a reactivity of LDL and the enzyme is suppressed by using calixarene, and a reactivity of HDL and the enzyme is suppressed by using albumin, both in the presence of calixarene and albumin is not disclosed.

JP-A-9 043 234 then describes an assay-reagent for CK-activity that originates from heparin, which is described as preventing turbidity when using a basic amino acid. However, said document does not describe an assay method for cholesterol in VLDL. Furthermore, a test for cholesterol in VLDL is not described, wherein a reactivity of LDL and the enzyme is suppressed by using calixarene, and a reactivity of HDL and an enzyme is suppressed by using albumin, both in the presence of calixarene and albumin.

### Disclosure of the Invention

An object of the present invention is to provide a method for a selective assay of components, particularly cholesterol, in VLDL, which is one of serum lipoproteins.

In view of such circumstances, the present inventors have carried out intensive investigations for a quantitative determination of the components in VLDL in biological samples, particularly in serum, using a commonly used automatic analysis apparatus without separation by means of centrifugal separation and without forming turbidity by a complex or aggregate in the reaction solution. As a result, they have found a method for assaying the VLDL-C without the formation of complex or aggregate which is causing turbidity causing the assay error **characterized in that** an enzymatic reaction by lipoprotein lipase (LPL) or cholesterol esterase (CE) dominantly and directly acting on the VLDL is carried out in the presence of at least calixarene or a salt thereof, particularly in the presence of at least calixarene or a salt thereof and albumin and/or a basic amino acid and, as a result, the present invention has been achieved.

Thus, the present invention comprises (1) A method for assaying cholesterol in a very low-density lipoprotein (VLDL) fraction by an enzymatic reaction, **characterized in that** an enzymatic reaction showing a high specificity to VLDL is carried out by addition of at least calixarene or a salt thereof and a component selected from the group consisting of a basic amino acid and albumin, wherein a reactivity of the enzyme with low-density lipoprotein is suppressed by the calixarene and a reactivity of the enzyme with high-density lipoprotein is suppressed by the component; (2) the method for assaying cholesterol in VLDL according to the above (1) wherein the enzymatic reaction is carried out by addition of at least the albumin, the basic amino acid, and the calixarene or the salt thereof; (3) the method for assaying cholesterol in VLDL according to the above (1) wherein the enzyme showing a high specificity to VLDL is lipoprotein lipase (LPL) and/or cholesterol esterase (CE) derived from *Chromobacterium* or *Pseudomonas*; (4) the method for assaying cholesterol in VLDL according to the above (1) wherein the enzymatic reaction for the assay of cholesterol uses cholesterol dehydrogenase and oxidized co-enzyme, and a reduced coenzyme, which is the product of the enzymatic reaction, is spectrometrically detected.; (5) the method for assaying cholesterol in VLDL according to any of the above (1) to (4) wherein the enzymatic reaction for the assay of cholesterol uses cholesterol oxidase, and hydrogen peroxide, which is the product of the enzymatic reaction, is spectrometrically detected using peroxidase; and (6) a reagent kit equipped with reagents necessary for conducting the method for assaying cholesterol in VLDL according to any of the above (1) to (5).

### Best Mode for Carrying Out the Invention

The present invention will now be illustrated in detail as hereunder.

With regard to an enzyme which acts on lipoprotein in order to hydrolyze it, whereby the components of lipoprotein are liberated, there are cholesterol ester hydrolases, such as LPL or CE. Among them, LPL or CE which is derived for example, from *Chromobacterium viscosum* or *Pseudomonas,* is preferably used, since it predominantly reacts with VLDL compared with other lipoproteins. Even an enzyme which is modified, for example, with the aim of stabilizing the enzyme may be used as long as it is an enzyme dominantly reacting with VLDL and thus being able to achieve the object of the present invention. Therefore, there is no particular limitation whether or not it is modified.

When calixarenes are added, reactivity of LPL and CE with LDL can be suppressed, and the substrate specificity of such an enzyme is improved. Calixarene suppresses the reactivity of LPL and CE with LDL when it is bound near the sugar chain and fatty acid of the surface of LDL. With regard to calixarenes, there are listed calixarene and a salt thereof such as calix[4]arene, calix[6]arene, calix[8]arene, calix[4]arene sulfate, calix[6]arene sulfate, calix[8]arene sulfate, calix[4]arene acetate, calix[6]arene acetate, calix[8]arene acetate, carboxycalix[4]arene, carboxycalix[6]arene, carboxycalix[8]arene, calix[4]arene amine, calix[6]arene amine and calix[8]arene amine. An appropriate one may be selected therefrom, and the concentration for the actual use may be decided upon by simple experimental repetition. One or more kind(s) of calixarene may be used for the method of testing according to the present invention.

In the method of testing according to the present invention, it is preferred to use albumin in addition to calixarenes. LPL or CE, having selectivity to VLDL, inherently act on HDL as well. Thus, LPL or CE shows a high reactivity with HDL and VLDL while it reacts weakly with LDL. When albumin is added, the reactivity of LPL or CE with HDL is suppressed whereupon the enzyme reacts only with VLDL. Bovine serum albumin is usually used as albumin, although there is no particular limitation for its origin. The actual concentration may be determined by experiments, and the range of 0.05∼10% is usable.

Similarly, it is possible to suppress the reaction with HDL using a basic amino acid. Thus, in the method of testing according to the present invention, it is possible to use a basic amino acid and calixarene together or to use calixarene and albumin together. It is more preferred in the present invention that albumin and a basic amino acid are used together in addition to calixarene.

With regard to a basic amino acid, usually arginine, arginine hydrochloride, lysine, lysine hydrochloride, histidine, histidine hydrochloride, hydroxylysine or hydroxylysine hydrochloride may be used, and they may be appropriately selected and used. The actual concentration may be determined by experiments and the range of 0.1∼1,000 mmol/L is usable. One or more kind(s) of basic amino acid may be used for the method of testing according to the present invention.

The component to be assayed in VLDL is cholesterol,and the testing of cholesterol is carried out in accordance with the common method for testing the total cholesterol. For example, there has been known a method where cholesterol is made to react with cholesterol dehydrogenase (CDH) and oxidized coenzyme, and the reduced coenzyme, which is a product of the reaction is spectrometrically detected. CDH requires hydrazine for potentiating the quantifying property of the reaction [an art of Japanese Patent Laid-Open No. 05/176,797 (December 27, 1991)] and is used in combination with other conditions such as activator and stabilizer. Known art may be applied therefore. For example, activators and stabilizers are nonionic surface-active agents and cholic acids. The actual concentration thereof may be determined by experiments. Examples of the oxidized coenzyme are oxidized β-nicotinamide adenine dinucleotide (NAD), oxidized thio-nicotinamide adenine dinucleotide (t-NAD), oxidized β-nicotinamide adenine dinucleotide phosphate (NADP) and oxidized thio-nicotinamide adenine dinucleotide phosphate (t-NADP).

It is also possible to use a method where cholesterol is made to react with cholesterol oxidase (COD) and hydrogen peroxide, which is a product of the reaction, is spectrometrically detected using peroxidase. With regard to a method for quantitative determination of hydrogen peroxide using peroxidase, known methods may be used. When the assay is carried out using COD, activators and stabilizers for enzyme are appropriately selected and used in the same manner as in the case of the assay using CDH. Conditions such as the actual concentration used may be determined by experiments.

The present invention further provides a reagent kit equipped with reagents necessary for carrying out the above-mentioned method for testing cholesterol in VLDL according to the present invention. The reagent kit of the present invention contains one or more substances selected at least from calixarenes, albumin and basic amino acids and is a reagent kit for the test of cholesterol in the manufactured VLDL.

### Examples

As hereinafter, a method for the test of cholesterol in VLDL will be illustrated in more detail by way of Examples although the present invention is not limited thereto.

### (Example 1)

The following reagents were prepared. With regard to the samples, 30 human serum examples from volunteers were used. The assay was carried out using an Automatic Analysis Apparatus, Type 7170, of Hitachi. First, 180 µL of the reagent 1 were added to 18 µL of the sample followed by keeping at 37°C for 5 minutes and, at this stage, absorbance 1 was measured at the dominant wavelength of 340 nm and non-dominant wavelength of 570 nm. Then, 60 µL of the reagent 2 were added followed by keeping at 37°C for 5 minutes and, at this stage, absorbance 2 was measured at the dominant wavelength of 340 nm, and non-dominant wavelength of 570 nm. The difference between the absorbance 1 and the absorbance 2 was calculated and converted to the amount of the sample using a control of known VLDL-cholesterol concentration as a standard solution.

The control method was in accordance with a method by Havel et al. [Have RJ, Eder HA, Bragdon JH: The distribution and chemical composition of ultracentrifugally separated lipoproteins in human serum, J. Clin. Invest., 34, 1345∼1353 (1955)]. Cholesterol in the fractionated VLDL fraction was determined by a T-CHO reagent L "Kokusai" (International Reagents Corporation). Table 1 shows the results of the test, using 30 samples of human serum. In the present method, the correlation coefficient was 0.993 (p < 0.05) and the result was in good agreement with the control method.

### Reagent 1

| | |
|---|---|
| HEPES buffer (pH 7.5) | 50 mmol/L |
| Hydrazinium dichloride | 60 mmol/L |
| Sodium cholate (activator) | 0.4% |
| Oxidized β-nicotinamide adenine dinucleotide | 6.0 mmol/L |
| Calix[8]arene sulfate | 10.0 mmol/L |
| Bovine serum albumin | 2.0% |
| Arginine | 200 mmol/L |

### Reagent 2

| | |
|---|---|
| HEPES buffer (pH 8.0) | 50 mmol/L |
| Cholesterol dehydrogenase | 20 U/mL |
| Cholesterol ester hydrolase (derived from *Pseudomonas* sp.) | 3 U/mL |
| Sodium cholate | 0.1% |

### (Example 2)

The following reagents were prepared. With regard to the samples, 10 human serum examples from normal healthy persons were used. The assay was carried out using an Automatic Analysis Apparatus, Type 7170, of Hitachi. First, 180 µL of the reagent 3 were added to 15 µL of the sample, followed by keeping at 37°C for 5 minutes and, at this stage, absorbance 1 was measured at the dominant wavelength of 600 nm and non-dominant wavelength of 700 nm. Then 60 µL of the reagent 4 were added followed by keeping at 37°C for 5 minutes and, at this stage, absorbance 2 was measured at the dominant wavelength of 360 nm, and non-dominant wavelength of 700 nm. The difference between the absorbance 1 and the absorbance 2 was calculated and converted to the amount of the sample using a control of known VLDL-cholesterol concentration as a standard solution. The control method used was the same as in Example 1. Table 2 shows the result of assay using 30 samples of human serum. In the present method, the correlation coefficient was 0.997 (p < 0.05) and the result was in good agreement with the control method.

### Reagent 3

| | |
|---|---|
| MES buffer (pH 6.5) | 50 mmol/L |
| HDAOS | 2.0 mmol/L |
| Emulgen 913 (activator) | 0.02% |
| Sodium cholate (activator) | 0.5% |
| Calix[8]arene sulfate | 10.0 mmol/L |
| Bovine serum albumin | 1.5% |
| Ascorbic acid oxidase | 3 U/mL |
| Arginine | 200 mmol/L |

### Reagent 4

| | |
|---|---|
| MES reagent (pH 6.5) | 50 mmol/L |
| Cholesterol oxidase | 2 U/mL |
| Cholesterol ester hydrolase derived from *Chromobacterium viscosum* | 3 U/mL |
| 4-Aminoantipyrine | 10 mmol/L |
| Sodium cholate | 0.1% |
| Peroxidase | 15 U/mL |

**Table 1 (unit: mg/dL)**

| Sample | Control method | Present method |
|---|---|---|
| 1 | 1.2 | 9.1 |
| 2 | 2.2 | 8.4 |
| 3 | 4.6 | 5.7 |
| 4 | 25.2 | 25.2 |
| 5 | 231.2 | 242.0 |
| 6 | 5.8 | 7.5 |
| 7 | 1.4 | 14.4 |
| 8 | 25.4 | 38.5 |
| 9 | 9.6 | 19.3 |
| 10 | 4.0 | 16.2 |
| 11 | 6.2 | 13.2 |
| 12 | 2.0 | 4.6 |
| 13 | 13.4 | 15.4 |
| 14 | 5.8 | 14.2 |
| 15 | 23.6 | 20.1 |
| 16 | 2.6 | 2.9 |
| 17 | 1.2 | 2.2 |
| 18 | 21.6 | 20.1 |
| 19 | 1.2 | 11.0 |
| 20 | 16.9 | 28.0 |
| 21 | 32.4 | 42.0 |
| 22 | 50.8 | 57.0 |
| 23 | 87.4 | 90.5 |
| 24 | 104.9 | 99.4 |
| 25 | 129.0 | 114.4 |
| 26 | 142.0 | 134.9 |
| 27 | 165.3 | 154. 8 |
| 28 | 45.1 | 56.2 |
| 29 | 31.3 | 40.1 |
| 30 | 23.1 | 35.7 |
| Correlation Coefficient | | 0.993 |
| Inclination of Regression Line | | 0.947 |
| Intercept of Regression Line | | 6.364 |

**Table 2 (unit: mg/dL)**

| Samples | Control Method | Present Method |
|---|---|---|
| 1 | 1.2 | 4.6 |
| 2 | 2.2 | 4.5 |
| 3 | 4.6 | 6.4 |
| 4 | 25.2 | 25.2 |
| 5 | 231.2 | 250.6 |
| 6 | 5.8 | 8.0 |
| 7 | 1.4 | 4.4 |
| 8 | 25.4 | 27.9 |
| 9 | 9.6 | 9.6 |
| 10 | 4.0 | 6.6 |
| 11 | 6.2 | 3.9 |
| 12 | 2.0 | 5.4 |
| 13 | 13.4 | 16.2 |
| 14 | 5.8 | 4.5 |
| 15 | 23.6 | 20.2 |
| 16 | 2.6 | 4.6 |
| 17 | 1.2 | 3.2 |
| 18 | 21.6 | 20.5 |
| 19 | 1.2 | 1.7 |
| 20 | 16.9 | 17.7 |
| 21 | 32.4 | 33.9 |
| 22 | 50.8 | 57.4 |
| 23 | 87.4 | 81.4 |
| 24 | 104.9 | 101.2 |
| 25 | 129.0 | 119.3 |
| 26 | 142.0 | 144.4 |
| 27 | 165.3 | 160.0 |
| 28 | 45.1 | 44.6 |
| 29 | 31.3 | 29.7 |
| 30 | 23.1 | 24.9 |
| Correlation Coefficient | | 0.997 |
| Inclination of Regression Line | | 1.012 |
| Intercept of Regression Line | | 0.398 |

### Industrial Applicability

The present invention as established provides a method for the testing of cholesterol in VLDL, which is one of serum lipoproteins and, since measurement of amount of the cholesterol in VLDL can be carried out selectively and easily, it is useful for clinical testing, investigation, etc. for diseases which are related to lipoprotein such as hyperlipemia.

## Claims

1. A method for assaying cholesterol in a very low-density lipoprotein (VLDL) fraction by an enzymatic reaction, **characterized in that** an enzymatic reaction showing a high specificity to VLDL is carried out by addition of at least calixarene or a salt thereof, and a component selected from the group consisting of a basic amino acid and albumin, wherein a reactivity of the enzyme with low-density lipoprotein is suppressed by the calixarene and a reactivity of the enzyme with high-density lipoprotein is suppressed by said component.

2. The method for assaying cholesterol in VLDL according to claim 1, wherein the enzymatic reaction is carried out by addition of at least the albumin, the basic amino acid, and the calixarene or the salt thereof.

3. The method for assaying cholesterol in VLDL according to claim 1 or claim 2, wherein the enzyme showing a high specificity to VLDL is lipoprotein lipase (LPL) and/or cholesterol esterase (CE) derived from *Chromobacterium* or *Pseudomonas.*

4. The method for assaying cholesterol in VLDL according to any of claims 1 to 3, wherein the enzymatic reaction for the assay of cholesterol uses cholesterol dehydrogenase and oxidized coenzyme, and a reduced coenzyme, which is the product of the enzymatic reaction, is spectrometrically detected.

5. The method for assaying cholesterol in VLDL according to any of claims 1 to 3, wherein the enzymatic reaction for the assay of cholesterol uses cholesterol oxidase, and hydrogen peroxide, which is the product of the enzymatic reaction, is spectrometrically detected using peroxidase.

6. A reagent kit for assaying cholesterol in a very low-density lipoprotein (VLDL) fraction by an enzymatic reaction, the reagent kit comprising a first reagent which contains calixarene or a salt thereof, and a component selected from the group consisting of a basic amino acid and albumin, and a second reagent which contains an enzyme selected from lipoprotein lipase (LPL) and/or cholesterol esterase (CE) derived from *Chromobacterium* or *Pseudomonas,* wherein a reactivity of the enzyme with the low-density lipoprotein is suppressed by the calixarene, and a reactivity of the enzyme with the high-density lipoprotein is suppressed by the component.

7. The reagent kit according to claim 6, wherein the first reagent contains oxidized coenzyme and the second reagent contains cholesterol dehydrogenase.

8. The reagent kit according to claim 6, wherein the second reagent contains cholesterol oxidase and peroxidase.

## Patentansprüche

1. Verfahren zum Testen auf Cholesterin in einer Fraktion von Lipoprotein sehr geringer Dichte (VLDL) mittels einer enzymatischen Reaktion, **dadurch gekennzeichnet, dass** eine enzymatische Reaktion, die eine hohe Spezifität für VLDL zeigt, durch Hinzugabe von mindestens Calixaren oder einem Salz davon und einer Komponente ausgewählt aus der Gruppe bestehend aus einer basischen Aminosäure und Albumin durchgeführt wird, wobei eine Reaktivität des Enzyms mit Lipoprotein geringer Dichte durch das Calixaren unterdrückt wird und eine Reaktivität des Enzyms mit Lipoprotein hoher Dichte durch die Komponente unterdrückt wird.

2. Verfahren zum Testen auf Cholesterin in VLDL nach Anspruch 1, wobei die enzymatische Reaktion durch Hinzugabe von mindestens dem Albumin, der basischen Aminosäure und dem Calixaren oder dem Salz davon durchgeführt wird.

3. Verfahren zum Testen auf Cholesterin in VLDL nach Anspruch 1 oder Anspruch 2, wobei das Enzym, das eine hohe Spezifität für VLDL zeigt, Lipoproteinlipase (LPL) und/oder Cholesterinesterase (CE), abgeleitet von *Chromobacterium* oder *Pseudomonas,* ist.

4. Verfahren zum Testen auf Cholesterin in VLDL nach einem der Ansprüche 1 bis 3, wobei die enzymatische Reaktion für den Test auf Cholesterin Cholesterindehydrogenase und oxidiertes Coenzym verwendet, und ein reduziertes Coenzym, das das Produkt der enzymatischen Reaktion ist, spektrometrisch nachgewiesen wird.

5. Verfahren zum Testen auf Cholesterin in VLDL nach einem der Ansprüche 1 to 3, wobei die enzymatische Reaktion für den Test auf Cholesterin Cholesterinoxidase verwendet, und das Wasserstoffperoxid, welches das Produkt der enzymatischen Reaktion ist, spektrometrisch unter der Verwendung von Peroxidase nachgewiesen wird.

6. Reagenzkit zum Testen auf Cholesterin in einer Fraktion von Lipoprotein sehr geringer Dichte (VLDL) mittels einer enzymatischen Reaktion, wobei das Reagenzkit ein erstes Reagenz umfasst, das Calixaren oder einem Salz davon enthält, und eine Komponente ausgewählt aus der Gruppe bestehend aus einer basischen Aminosäure und Albumin, und ein zweites Reagenz, das ein Enzym ausgewählt aus Lipoproteinlipase (LPL) und/oder Cholesterinesterase (CE), abgeleitet von *Chromobacterium* oder *Pseudomonas,* enthält, wobei eine Reaktivität des Enzyms mit Lipoprotein geringer Dichte durch das Calixaren unterdrückt wird und eine Reaktivität des Enzyms mit Lipoprotein hoher Dichte durch die Komponente unterdrückt wird.

7. Reagenzkit nach Anspruch 6, wobei das erste Reagenz oxidiertes Coenzym enthält und das zweite Reagenz Cholesterindehydrogenase enthält.

8. Reagenzkit nach Anspruch 6, wobei das zweite Reagenz Cholesterinoxidase und Peroxidase enthält.

## Revendications

1. Procédé d'analyse du cholestérol dans une fraction de lipoprotéine à très basse densité (VLDL) par une réaction enzymatique, **caractérisé en ce qu'**une réaction enzymatique montrant une spécificité élevée à la VLDL est réalisée par addition d'au moins du calixarène ou un sel de celui-ci, et d'un composant choisi dans le groupe constitué d'un acide aminé basique et d'albumine, dans lequel une réactivité de l'enzyme avec une lipoprotéine à faible densité est supprimée par le calixarène et une réactivité de l'enzyme avec une lipoprotéine à haute densité est supprimée par ledit composant.

2. Procédé d'analyse du cholestérol dans la VLDL selon la revendication 1, dans lequel la réaction enzymatique est réalisée par addition d'au moins l'albumine, l'acide aminé basique et le calixarène ou son sel.

3. Procédé d'analyse du cholestérol dans la VLDL selon la revendication 1 ou la revendication 2, dans lequel l'enzyme présentant une spécificité élevée à la VLDL est la lipoprotéine lipase (LPL) et/ou une cholestérol-estérase (CE) dérivée de *Chromobacterium* ou *Pseudomonas*.

4. Procédé d'analyse du cholestérol dans la VLDL selon l'une quelconque des revendications 1 à 3, dans lequel la réaction enzymatique pour l'analyse de cholestérol utilise de la déshydrogénase de cholestérol et une coenzyme oxydée, et une coenzyme réduite, qui est le produit de la réaction enzymatique, est détectée de manière spectrométrique.

5. Procédé d'analyse du cholestérol dans la VLDL selon l'une quelconque des revendications 1 à 3, dans lequel la réaction enzymatique pour l'analyse de cholestérol utilise l'oxydase de cholestérol et le peroxyde d'hydrogène, qui est le produit de la réaction enzymatique, est détecté de manière spectrométrique en utilisant la peroxydase.

6. Kit de réactif pour analyser le cholestérol dans une fraction de lipoprotéine à très basse densité (VLDL) par une réaction enzymatique, le kit de réactif comprenant un premier réactif qui contient du calixarène ou son sel, et un composant choisi dans le groupe constitué d'un acide aminé basique et d'albumine, et un second réactif qui contient une enzyme choisie parmi la lipoprotéine lipase (LPL) et/ou la cholestérol estérase (CE) dérivée de *Chromobacterium* ou *Pseudomonas,* dans lequel une réactivité de l'enzyme avec la lipoprotéine basse densité est supprimée par le calixarène, et une réactivité de l'enzyme avec la lipoprotéine haute densité est supprimée par le composant.

7. Kit de réactif selon la revendication 6, dans lequel le premier réactif contient une coenzyme oxydée et le second réactif contient de la déshydrogénase de cholestérol.

8. Kit de réactif selon la revendication 6, dans lequel le second réactif contient de l'oxyde et de la peroxydase de cholestérol.
